# EUROPEAN PATENT APPLICATION

(11) **EP 4 610 990 A1**
(43) Date of publication of application: **03.09.2025**
(21) Application number: 24182777.3
(22) Date of filing: 18.06.2024
(51) Int. Cl.: G16H 10/20, A61B 5/00, G06N 3/08, G06N 20/00, G16H 50/20, G16H 50/30, G16H 50/70

(54) **A TOOTH DECAY RISK ASSESSMENT SYSTEM**

(30) Priority: 27.02.2024 PT 2024119275; 11.06.2024 PT 2024119522
(71) Applicant: Soares Guerreiro, Eduardo Manuel, 7630-017 Almograve (PT)
(72) Inventor: Soares Guerreiro, Eduardo Manuel, 7630-017 Almograve (PT)
(74) Representative: do Nascimento Gomes, Rui

(57) **Abstract**

It is described tooth decay risk assessment system, to provide a constant and personalized communication channel between patients and medical staff, allowing real-time monitoring of the patient's oral health status and enabling early interventions based on risk analysis. The system may be implemented through a wireless communication network (4) that interconnects a computing platform (1) accessible through a mobile application by both patient and medical staff processing devices (2, 3), for example of the smartphone type. The system offers continuous and detailed monitoring, supported by artificial intelligence capabilities for data analysis as a way to provide dynamic, more accurate and personalized tooth decay risk assessments.

## Description

### FIELD OF THE APPLICATION

The present application relates generally to systems adapted to identify an oral condition or disease. More particularly, the present application belongs to the field of systems that employ computational capabilities for identifying an oral condition or disease and for generating a treatment plan for the identified oral condition or disease.

### PRIOR ART

Traditional risk management tools for tooth decay often suffer from significant limitations, primarily due to their one-off nature and lack of customization. They are often based on static assessments that cannot keep up with dynamic changes in patients' oral health habits and conditions over time.

The present solution intended to innovatively overcome such issues.

### SUMMARY OF THE APPLICATION

It is therefore an object of the present application a tooth decay risk assessment system, for performing tooth decay risk management. Such system, as described herein, overcomes state-of-the-art limitations by offering continuous and detailed monitoring, supported by artificial intelligence capabilities for data analysis as a way to provide dynamic, more accurate and personalized tooth decay risk assessments.

In this way, it is possible to accurately assess the probability of the occurrence of tooth decay, which takes into account the dynamic nature of this disease that is imposed by the habits of each patient. In effect, the system developed is designed to carry out a dynamic and personalized risk assessment, facilitating early diagnosis and allowing management of the treatment process adaptable to each patient. Consequently, the system allows identifying the need to carry out medical interventions in a timely and evidence-based manner, making it technically more advantageous than state-of-the-art systems.

In an advantageous configuration of the system, it is comprised by one or more processing units operatively coupled to each other and configured to:
- Receive patient data related to a patient;
- Execute a first AI model configured to determine a patient's oral status based upon the received patient data;
- Generate dental questionnaires based upon at least the patient's oral status and providing said questionnaires to the patient;
- Receive patient inputs in response to questionnaires;
- Execute a second AI model configured to generate AI prediction on tooth decay risk, to determine a tooth decay risk assessment value based upon at least the received patient data and patient inputs.

It therefore provides a better mode of understanding the dynamics of oral health, which is very important for establishing new standards in prevention and treatment of tooth decay. In fact, the system uses artificial intelligence to analyse collected data to improve risk identification and suggest preventive measures, ensuring a proactive approach to oral health.

### DESCRIPTION OF FIGURES

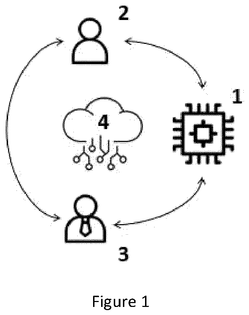

Figure 1- representation of an embodiment of the tooth decay risk assessment system. The numerical reference represent:
1- computing platform;
2- patient's processing device;
3- medical staff's processing device;
4-wireless communication network.

### DETAILED DESCRIPTION

A computer system configured to execute a new methodology for managing oral health is proposed, making it more proactive, personalized and effective in identifying and treating tooth decay. Its implementation therefore allows for continuous improvement in oral health care and promotes a greater quality of life for patients.

Through the use of artificial intelligence, in particular the execution of artificial intelligence models, for example deep learning models, which allow advanced predictive reasoning to be carried out, the probability of the patient developing new tooth decay is assessed, considering the data that are provided by the patients, for example in response to periodic questionnaires sent to them, and by the medical staff, for example, a dentist. Furthermore, it allows personalized preventive measures to be adopted for each patient.

The system was developed to provide a constant and personalized communication channel between patients and medical staff, allowing real-time monitoring of the patient's oral health status and enabling early interventions based on risk analysis. Figure 1 illustrates a schematic realization of a system's embodiment, that is implemented through a wireless communication network (4) that interconnects a computing platform (1) accessible through a mobile application by both patient and medical staff processing devices (2, 3), for example of the smartphone type.

In a preferred and advantageous embodiment of the tooth decay risk assessment system described in the present application, it is comprised by one or more processing units that are operatively connected to each other. This means that the system may comprise only one processing unit, or a plurality of processing units, which in this case requires them to be connected to each other, for the purpose of sharing and processing data, in order to guarantee the operation of the system. More particularly, the one or more processing units are configured to execute the following set of actions:
In a first action, the one or more processing units are configured to receive patient data related to a patient. Said patient data may related to, for example, patient's gender and age, number of dental caries and dental restorations, date of previous dental restoration or salivary flow tests.

In a second action, the one or more processing units are configured to execute a first AI model, in order to determine a patient's oral status based upon the received patient data. In the context of the present application, a patient's oral status and is determined based upon patient's habits and oral health related, among others, with oral hygiene, level of fluoride and the patient's medical and dental history. In this context, the first AI model being configured to determine a patient's oral status means that it is trained with historical data to learn patterns and complex relationships between variables, from which it uses artificial intelligence algorithms to determine patient's oral status and to improve the accuracy of such determinations.

In a third and fourth actions, the one or more processing units are configured to generate dental questionnaires based upon at least the patient's oral status and providing said questionnaires to the patient, and to receive patient inputs in response to questionnaires. A dental questionnaire is formed by a set of questions that are directed to a patient based on his oral status, providing a dynamic tooth decay risk assessment over time, which facilitates early diagnosis and personalization of treatment. The questionnaire may include multiple sections relating to general patient data (such as patient's medical history, dental history, social history, general tooth decay risk factors and current symptoms being presented by the patient, e.g. fever, bad breath, bleeding gums, generalised pain etc) and more specific dental issues (can include a representation of populated dental chart indicating the tooth number applicable to said dental issue, the location of the dental issue at the surface level or tooth level, and one or more questions related to the symptoms of the dental issue. Each section may include a pre-set of questions depend on a particular patient condition or dental issue. In the context of the embodiment of the system illustrated by Figure 1, the dental questionnaire may be presented to the patient's smartphone (2) via a mobile application graphical user interface or a computing platform's (1) dashboard.

Finally, in a fifth action, the one or more processing units are configured to execute a second AI model, in order to determine a tooth decay risk assessment value based upon at least the received patient data and patient inputs to the questionnaires. In the context of the present application, a tooth decay risk assessment value relates to a patient's risk level of contracting tooth decay. In this context, the second AI model being configured to determine a tooth decay risk assessment value means that it is trained with historical data to learn patterns and complex relationships between variables, from which it uses artificial intelligence algorithms to determine tooth decay risk value and to improve the accuracy of such determinations.

In this way, the system provides an understanding of the dynamics of oral health, which is very important for establishing new standards in the prevention and treatment of tooth decay. In fact, by using artificial intelligence, for example deep learning models, to analyse collected data, it is possible to improve risk identification and suggest preventive measures, ensuring a proactive approach to oral health.

In one embodiment of the system, the one or more processing units are further configured to transmit the tooth decay risk assessment value to the patient. Additionally, the one or more processing unit may be further configured to generate a tooth decay risk assessment report based upon the tooth decay risk assessment value and to transmit said report to the patient. Through the wireless communication network (1) architecture that supports the system's embodiment illustrated in figure 1, the tooth decay risk assessment value or the corresponding report can be transmitted or consulted directly by patients and medical staff through their smartphone (2, 3) and the mobile application that runs on the said processing device (2, 3) and which establishes a user interface with the computing platform (1) that provides that information.

In another embodiment of the system, it further comprises an oral patient server database adapted to store patient oral care data related to patients. More particularly, said server database is operatively connected to the one or more processing units, so that the one or more processing units are further configured to establish the following actions:
In a first action, the one or more processing units are further configured to populate the oral patient server database with patient data and patient inputs. In the context of the present application, populate a server database should be understood as including all computational actions adapted to filling one or more tables with specific data. Subsequently, in a second action, the one or more processing unit are configured to generate dental questionnaires based upon the populated oral patient server database.

In another advantageous embodiment of the system, the one or more processing units are configured to generate dental questionnaires and providing said questionnaires to the patient on a periodic basis, over an assessment period which may be preconfigurable. Said assessment period is the time during which the patient's tooth decay risk assessment value is to be calculated, that is, it is the period of time during which the particular configuration of processing unit actions shall be executed in order to determine the tooth decay risk assessment value. Said assessment period may be 30 days during which the one or more processing units are configured to provide dental questionnaires to the patient on a daily basis. In this way, a dynamic and personalized risk assessment is carried out, through continuous monitoring of patients' habits, which allows the prevention of tooth decay, aligned with a long-term vision for oral health, establishing a standard of care proactive, allowing to facilitate early diagnosis.

In another advantageous embodiment of the system, the one or more processing units are further configured to generate a treatment plan based on at least the tooth decay risk assessment value and to transmit the treatment plan to the patient. More particularly, the one or more processing units may be further configured to generate the treatment plan further based on patient inputs in response to questionnaires. In this way, a dynamic and personalized risk assessment is carried out, through continuous monitoring of patients' habits, which allows the prevention of tooth decay, aligned with a long-term vision for oral health, establishing a standard of care proactive and personalization of treatment. Through the wireless communication network (1) architecture that supports the system's embodiment illustrated in figure 1, the treatment plan may be transmitted or consulted directly by patients through their smartphone (2) and the mobile application that runs on the said processing device (2) and which establishes a user interface with the computing platform (1) that provides that information.

In another advantageous embodiment of the system, it further comprises at least one server computer operable to receive and store a plurality of user profiles and to attribute to each user profile an Entity type. More particularly, an Entity type may relate to at least a patient entity type having a respective permission level configured to allow a user to provide respective patient data, patient inputs to questionnaires and to access to information related to tooth decay risk assessment values, tooth decay risk assessment reports and treatment plans. Additionally, an Entity type may further relate to a medical staff entity type having a respective permission level configured to at least allow a user to provide expert data to generate dental questionnaires and treatment plans and to access to information related to user inputs to questionnaires, tooth decay risk assessment values, tooth decay risk assessment reports and treatment plans. In this context, the system may further comprise:
at least one processing device (2) assignable to a patient entity type user profile, provided with input means and comprising a communication unit configured to establish a bidirectional communication link (4) with the one or more processing units; and
at least one processing device (3) assignable to a medical staff entity type user profile, provided with input means and comprising a communication unit configured to establish a bidirectional communication link (4) with the one or more processing units and with the one or more processing devices (2) assignable to a patient type entity user profile.

As will be clear to one skilled in the art, the present invention should not be limited to the embodiments described herein, and a number of changes are possible which remain within the scope of the present invention.

Of course, the preferred embodiments shown above are combinable, in the different possible forms, being herein avoided the repetition all such combinations.

## Claims

1. A tooth decay risk assessment system comprising one or more processing units operatively connected to each other and configured to:
- Receive patient data related to a patient;
- Execute a first AI model configured to determine a patient's oral status based upon the received patient data;
- Generate dental questionnaires based upon at least the patient's oral status and providing said questionnaires to the patient;
- Receive patient inputs in response to questionnaires;
- Execute a second AI model configured to generate AI prediction on tooth decay risk, to determine a tooth decay risk assessment value based upon at least the received patient data and patient inputs.

2. System according to claim 1, wherein the one or more processing units are further configured to:
- Transmit the tooth decay risk assessment value to the patient.

3. System according to claim 1, wherein the one or more processing units are further configured to:
- Generate a tooth decay risk assessment report based upon the tooth decay assessment value; and
- Transmit the tooth decay risk assessment report to the patient.

4. System according to any of the previous claims, wherein patient data relates to at least:
- patient's gender and age;
- number of dental caries and dental restorations,
- date of previous dental restoration and
- salivary flow tests.

5. System according to any of the previous claims further comprising an oral patient server database for storing patient oral care data related to patients; said server database being operatively connected to the one or more processing units, so that the one or more processing units are further configured to:
- populate said oral patient server database based on patient data and patient inputs;
- generate dental questionnaires based upon the populated oral patient server database.

6. System according to any of the previous claims, wherein the one or more processing units are configured to:
- Generate dental questionnaires and providing said questionnaires to the patient on a periodic basis, over an assessment period.

7. System according to claim 6, wherein the assessment period is preconfigurable; optionally, the assessment period is 30 days.

8. System according to claims 6 or 7, wherein the one or more processing units are configured to provide dental questionnaires to the patient on a daily basis.

9. System according to any of the previous claims, wherein the one or more processing units are further configured to:
- Generate a treatment plan based on at least the tooth decay risk assessment value; and
- Transmit the treatment plan to the patient.

10. System according to claim 9, wherein the one or more processing units are configured to generate the treatment plan further based on patient inputs in response to questionnaires.

11. System according to any of the previous claims further comprising at least one server computer operable to:
- receive and store a plurality of user profiles;
- attribute to each user profile an Entity type; wherein an Entity type relates to at least a patient entity type having a respective permission level configured to allow a user to provide respective patient data, patient inputs to questionnaires and to access to information related to tooth decay risk assessment values, tooth decay risk assessment reports and treatment plans;
and wherein,
the system further comprising:
- At least one processing device assignable to a patient entity type user profile, provided with input means and comprising a communication unit configured to establish a bidirectional communication link with the one or more processing units.

12. System according to claim 11, wherein an Entity type further relates to a medical staff entity type having a respective permission level configured to at least allow a user to provide expert data to generate dental questionnaires and treatment plans and to access to information related to user inputs to questionnaires, tooth decay risk assessment values, tooth decay risk assessment reports and treatment plans;
and wherein,
the system further comprising:
- At least one processing device assignable to a medical staff entity type user profile, provided with input means and comprising a communication unit configured to establish a bidirectional communication link with the one or more processing units and with the one or more processing devices assignable to a patient type entity user profile.

13. System according to any of the previous claims, further comprising a wireless communication network adapted to operatively connect at least the one or more processing units.

14. System according to claims 11 to 13, wherein the wireless communication network is adapted to further connects processing devices assignable to patient and medical staff entity type user profiles.

15. System according to any of the previous claims, wherein the first and second AI models are deep learning models.
